(19) Europäisches Patentamt
European Patent Office
Office européen des brevets

(11) EP 1 085 810 B1

(12) EUROPÄISCHE PATENTSCHRIFT

(45) Veröffentlichungstag und Bekanntmachung des Hinweises auf die Patenterteilung:
**28.12.2005 Patentblatt 2005/52**

(21) Anmeldenummer: **99927800.5**

(22) Anmeldetag: **29.05.1999**

(51) Int Cl.[7]: **A01N 47/44**, B27K 3/50
// (A01N47/44, 43:836, 43:36, 43:54, 37:18, 43:28, 43:707), A01N37:44

(86) Internationale Anmeldenummer:
**PCT/EP1999/003739**

(87) Internationale Veröffentlichungsnummer:
**WO 1999/063826 (16.12.1999 Gazette 1999/50)**

(54) **MITTEL ZUR BEKÄMPFUNG VON PFLANZENSCHÄDLINGEN**
AGENTS FOR COMBATTING PLANT PESTS
AGENTS POUR LUTTER CONTRE DES PARASITES DE VEGETAUX

(84) Benannte Vertragsstaaten:
**AT BE CH DE DK ES FR GB IT LI NL PT SE**

(30) Priorität: **10.06.1998 DE 19825891**
**30.06.1998 DE 19829113**

(43) Veröffentlichungstag der Anmeldung:
**28.03.2001 Patentblatt 2001/13**

(60) Teilanmeldung:
**05016734.5 / 1 593 307**

(73) Patentinhaber: **Bayer CropScience AG**
**40789 Monheim (DE)**

(72) Erfinder:
- **ERDELEN, Christoph**
  **D-42799 Leichlingen (DE)**
- **ANDERSCH, Wolfram**
  **D-51469 Bergisch Gladbach (DE)**
- **STENZEL, Klaus**
  **D-40595 Düsseldorf (DE)**
- **MAULER-MACHNIK, Astrid**
  **D-42799 Leichlingen (DE)**
- **KRÄMER, Wolfgang**
  **D-51399 Burscheid (DE)**

(56) Entgegenhaltungen:
**WO-A-96/03045** **WO-A-97/24032**

- **DATABASE CAPLUS [Online] Accession no. 1993:228245, Document no. 118:228245, Nippon Soda Co.: "Synergistic agrochemical pesticide compositions containing amines and ergosterol biosynthesis inhibitors" XP002900600 & JP 05 017311 A 26. Januar 1993 (1993-01-26)**
- **DATABASE CAPLUS [Online] Accession no. 1992:607190, Document no. 117:207190, Takeda Yakuhin Kogyo K.K.: "Insecticidal and fungicidal compositions containing guanidines" XP002900601 & JP 04 108704 A**
- **DATABASE CAPLUS [Online] Accession no. 1993:488888, Document no. 119:88888, Takeda Chemical Industries, Ltd.: "Agrochemical compositions containing condensed heterocycle-containing amides and other active ingredients" XP002900602 & JP 05 039205 A 19. Februar 1930 (1930-02-19)**
- **DATABASE CAPLUS [Online] Accession no. 1992:545353, Document no. 117:145353, Takeda Chemical Industries, Ltd.: "Synergistic insecticide compositions containing guanidines and organophosphates" XP002900603 & JP 04 112805 A 14. April 1992 (1992-04-14)**
- **DATABASE CAPLUS [Online] Accession no. 1992:545352, Document no. 117:145352, Takeda Chemical Industries, Ltd.: "Synergistic insecticide compositions containing guanidines and carbamates" XP002900604 & JP 04 112804 A 14. April 1992 (1992-04-14)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **DATABASE CAPLUS [Online] Accession no. 1992:526474, Document no. 117:126474, Takeda Chemical Industries, Ltd.: "Synergistic insecticides containing guanidine derivatives" XP002900605 & JP 04 120007 A 21. April 1992 (1992-04-21)**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft Schädlingsbekämpfungsmittel die eine Wirkstoffkombination der Verbindung der Formel (I)

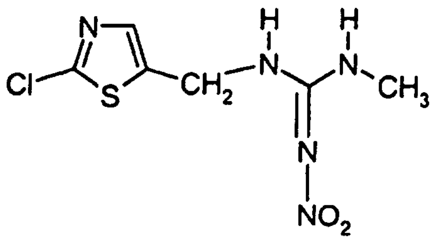

(I)

mit Fungiziden enthalten.

Fungizide Wirkstoffe, wie Azol-Derivate, Arylbenzylether, Benzamide, Morpholin-Verbindungen und andere Heterocyclen sind bekannt (vgl. K.H. Büchel "Pflanzenschutz und Schädlingsbekämpfung", Seiten 140 bis 153, Georg Thieme-Verlag, Stuttgart 1977, EP-OS 0 040 345, DE-OS 2 324 010, DE-OS 2 201 063, EP-OS 0 112 284, EP-OS O 304 758 und DD-PS 140 412).

Mischungen bestimmter Nitromethylenderivate mit fungiziden Wirkstoffen und ihre Verwendung als Schädlingsbekämpfungsmittel im Pflanzenschutz sind bereits bekannt (US-P-4 731 385; JP-OS 63-68507, 63/68505; 63/72 608; 63/72 609, 63/72 610, WO 96/03 045, JP 08 245 323, JP 04 368 303, JP 05 017 311, WO 97/22 254, WO 92/21 241). Mischungen bestimmter offenkettiger Nitromethylene und Nitroguanidine mit Fungiziden sind bereits bekannt (JP-OS 30 47 106; US-P 5 181 587).

In JP 0410 8704, WO 97/24032, JP 0503 9205, JP 0411 2805 und JP 0412 0007 werden Mischungen der Verbindung der Formel (I) mit anderen Wirkstoffen, darunter auch bestimmten Fungiziden, beschrieben.

Mischungen von Cyclopropylcarboxamiden mit bestimmten Nitromethylenen- oder Nitroguanidinderivaten sind bereits bekannt (JP-OS 3 271 207).

Mischungen von u.a. Imidacloprid und fungiziden Wirkstoffen zur Anwendung im Materialschutz und gegen Termiten, nicht aber zur Anwendung gegen pflanzenschädigende Schädlinge sind bereits bekannt (EP-OS 0 511 541). Mischungen von Imidacloprid und Azolylmethylcycloalkanen insbesondere Triticonazol sind bekannt aus EP-OS 545 834.

Es ist jedoch noch nichts darüber bekannt geworden, dass sich Nitroguanidinderivate und bestimmte Azolderivate in ihrer Wirkung gegenseitig so günstig beeinflussen, daß sie bei guter Pflanzenverträglichkeit in hervorragender Weise als Bekämpfungsmittel gegen Pflanzenschädlinge eingesetzt werden können.

Die vorliegende Erfindung betrifft Mittel gegen Pflanzenschädlinge, die die Verbindung der Formel (I)

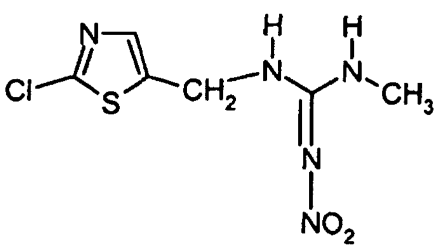

(I)

in Mischung mit unten genannten fungiziden Wirkstoffen enthalten.

Als Fungizide in den erfindungsgemäßen Mitteln zur Bekämpfung von Pflanzenschädlingen seien genannt:

(I) Azol-Derivate der Formel

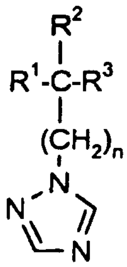

(II)

(II-1) $R^1$ = 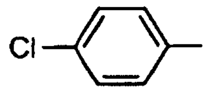 $(CH_2)_2$-, $R^2$ = -$C(CH_3)_3$, $R^3$ = OH, N = 1,

(TEBUCONAZOLE)

(II-3) $R^1$ = 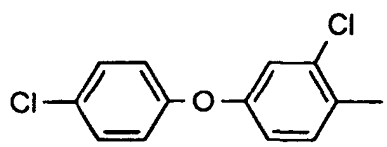 , n = 1,

$R^2$, $R^3$ = -$OCH_2CH(CH_3)O$-,

(DIFENCONAZOLE)

(II-4) $R^1$ = 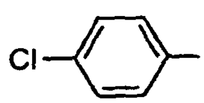 , $R^2$ = 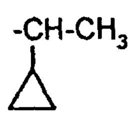 , $R^3$ = -OH, n = 1,

(CYPROCONAZOLE)

(II-5) $R^1$ = 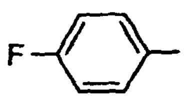 , $R^2$ = 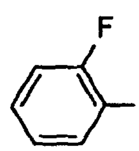 , $R^3$ = OH, n = 1,

(FLUTRIAFOL)

(II-6) $R^3$ = 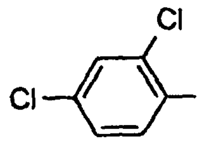 , $R^4 = -(CH_2)_3CH_3$, $R^5$ = OH, n = 1,

(HEXACONAZOLE)

(II-7) $R^1$ = 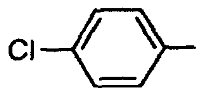 , $R^2 = -(CH_2)_3CH_3$, $R^3$ = CN, n = 1,

(MYCLOBUTANIL)

(II-8) $R^1$ = 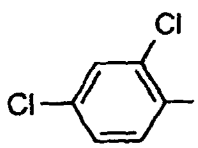 , $R^2 = -(CH_2)_2CH_3$, $R^3$ = H, n = 1,

(PENCONAZOLE)

(II-9) $R^1$ = 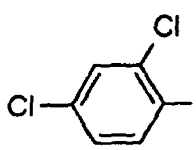 , $R^2$, $R^3$ = 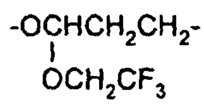 , n = 1,

(FURCONAZOLE)

(II-10) $R^1$ = 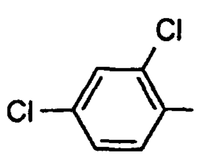 , $R^2$, $R^3$ = 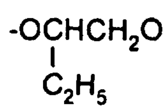 , n = 1,

(ETACONAZOLE)

(II-11) $R^1$ = 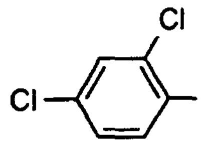 , $R^2$, $R^3$ = 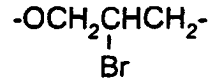 , n = 1,

(BROMUCONAZOLE)

(II-12) $R^1$ = 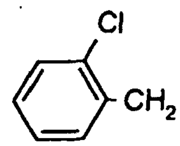 , $R^2$ = 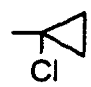 , $R^3$ = OH, n = 1,

(II-13) $R^1$ = 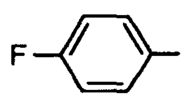 , $R^2$, $R^3$ = 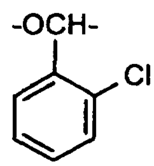 , n = 1,

(II-14) $R^1$ = 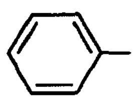 , $R^2$ = 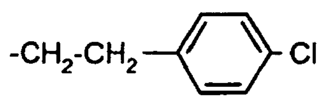 , n = 1, $R^3$ = CN,

(FENBUCONAZOLE)

(II-15) $R^1$ = 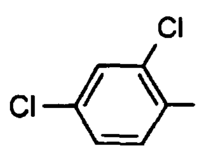 , $R^2$ = $CH_2OCF_2CHF_2$, $R^3$ = H, n = 1,

(TETRACONAZOLE)

(II-16) $R^1$ = 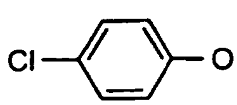 , $R^2$ = $-CH(OH)-C(CH_3)_3$, n = 0, $R^3$ = H,

(TRIADIMENOL)

(II-17) $R^1$ = 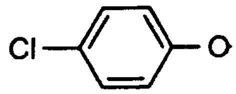, $R^2$ = $-CO-C(CH_3)_3$, n = 0, $R^3$ = H,

(TRIADIMEFON)

(II-19) $R^1$ = 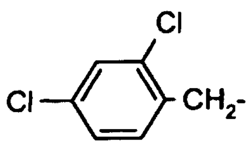, $R^2$ = $-CH(OH)-C(CH_3)_3$, n = 0, $R^3$ = H,

(DICLOBUTRAZOL)

(II-20) $R^1$ und $R^2$ = 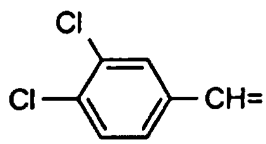, $R^3$ = 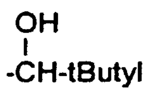, n = 0,

(DINICONAZOL)

Der Wirkstoff der Formel (I) ist aus EP-OS 0 375 907 bekannt.

Die fungiziden Wirkstoffe sind ebenfalls bekannt.

So werden z.B. in folgenden Publikationen beschrieben:

(1) Verbindungen der Formel (II)

DE-OS 2 201 063
DE-OS 2 324 010
DE-OS 2 737 489
DE-OS 3 018 866
DE-OS 2 551 560
EP 47 594
DE 2 735 872

Die erfindungsgemäßen Wirkstoffkombinationen enthalten neben dem Wirkstoff der Formel (I) mindestens einen fungiziden Wirkstoff ausgewählt aus den Verbindungen der Gruppe (1). Sie können darüber hinaus auch weitere Wirkstoffe sowie übliche Hilfs- und Zusatzstoffe sowie Verdünnungsmittel enthalten.

Als bevorzugte fungizide Wirkstoffe in den erfindungsgemäßen Mitteln seien genannt: Tebuconazol, Triadimenol, Cyproconazol.

Wenn die Wirkstoffe in den erfindungsgemäßen Wirkstoffkombinationen in bestimmten Gewichtsverhältnissen vorhanden sind, zeigt sich ein deutlicher synergistischer Effekt der Mischungen. Jedoch können die Gewichtsverhältnisse der Wirkstoffe in den Wirkstoffkombinationen in einem relativ großen Bereich variiert werden. Im allgemeinen entfallen auf 1 Gewichtsteil an Wirkstoff der Formel (I)

0,1 bis 10 Gewichtsteile, vorzugsweise
0,3 bis 3 Gewichtsteile an mindestens einem fungiziden Wirkstoff aus der Gruppe (1).

Die erfindungsgemäßen Wirkstoffkombinationen besitzen sehr gute fungizide Eigenschaften. Sie lassen sich vor allem zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Wirkstoffkombinationen eignen sich besonders gut zur Bekämpfung von Getreidekrankheiten, wie Erysiphe, Cochliobolus, Septoria, Pyrenophora und Leptosphaeria, und gegen Pilzbefall an Gemüse, Wein und Obst, beispielsweise gegen Venturia oder Podosphaera an Äpfeln, Uncinula an Reben oder Sphaeroteca an Gurken.

Die Wirkstoffkombinationen eignen sich auch sehr gut zur Bekämpfung von tierischen Schädlingen, vorzugsweise Arthropoden, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis porni, Eriosoma lanigerum, Hyalopterus arundinis, Macrosiphum avenae, Myzus spp., Phorodon humuli, Rhopalosiphum padi, Phylloxera vastatrix, Pemphigus spp., Empoasca spp., Euscelis bilobatus, Nephotettix cincticeps, Lecanium corni, Saissetia oleae, Laodelphax striatellus. Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor. Agriotes spp., Conoderus spp., Melolontha melolontha, Amphimallon solstitialls, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala. Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffkombinationen können in die üblichen Formulierungen übergeführt werden,

wie Lösungen, Emulsionen. Suspensionen, Pulver, Schäume, Pasten, Granulate, Aerosole, Feinstverkapselungen in polymeren Stoffen und in Hüllmassen für Saatgut, sowie ULV-Fonnulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffkombinationen können in den Formulierungen in Mischung mit anderen bekannten Wirkstoffen vorliegen, wie Fungizide, Insektizide, Akarizide und Herbizide, sowie in Mischungen mit Düngemitteln oder Pflanzenwachstumsregulatoren.

Die Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.

Die Anwendung geschieht in üblicher Weise, z.B. durch Gießen, Verspritzen, Versprühen, Verstreuen, Verstreichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Außerdem wurde gefunden, daß die erfindungsgemäßen Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticomis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie
Sirex juvencus, Urocerus gigas, Urocerus gigas taignus, Urocerus augur.

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergierbzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide enthalten.

Als zusätzliche Zumischpartner kommen vorzugsweise die in der Wo 94/29 268 genannten Insektizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron genannt.

Die gute pestizide Wirkung der erfindungsgemäßen Wirkstoffkombinationen geht aus den nachfolgenden Beispielen hervor. Während die einzelnen Wirkstoffe oder die bekannten Wirkstoffkombinationen in der pestiziden Wirkung Schwächen aufweisen, geht aus den Tabellen der folgenden Beispiele eindeutig hervor, daß die gefundene Wirkung der erfindungsgemäßen Wirkstoffkombinationen größer ist als Summe der Wirkungen der einzelnen Wirkstoffe und auch größer als die Wirkungen der bekannten Wirkstoffkombinationen.

In den folgenden Beispielen wird der Wirkstoff der Formel (I)

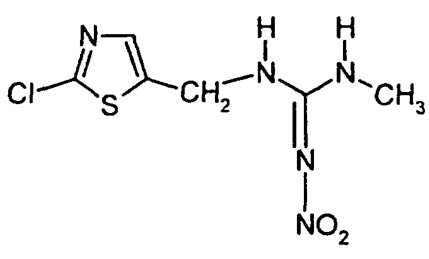

(I)

eingesetzt.

Die ebenfalls eingesetzten fungiziden Wirkstoffe werden in den Beispielen angegeben.

**Beispiel A**

| Plutella-Test | |
|---|---|
| Lösungsmittel | 7 Gewichtsteile Dimethylformamid |
| Emulgator | 1 Gewichtsteil Alkyarylpolyglykolether |

Zur Herstellung einer zweckmäßigen Wirkstoffzubereitung vermischt man 1 Gewichtsteil eines Wirkstoffes oder einer Wirkstoffkombination mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Wirkstoffkombinationen gemäß vorliegender Anmeldung eine synergistisch verstärkte Wirksamkeit im Vergleich zu den einzeln angewandten Wirkstoffen:

**Tabelle A**

(pflanzenschädigende Insekten)

**Plutella-Test**

| Wirkstoff/Wirkstoffgemisch | Wirkstoff/Wirkstoffgemisch-konzentration in % | Abtötungsgrad in % |
|---|---|---|
| 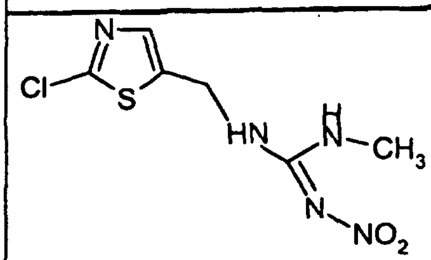 gemäß Formel (I) | 0.0008 | 0 |
| 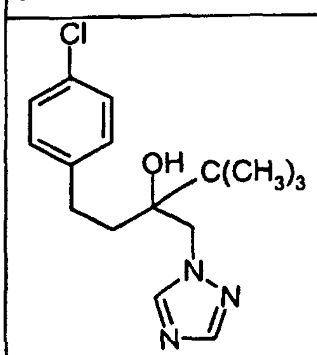 Tebuconazol | 0.02 | 0 |
| Formel (I) + Tebuconazol | 0.0008 + 0.02 | 100 |

**Beispiel B**

| Grenzkonzentrationstest/Bodeninsekten | |
|---|---|
| Testinsekt | Spodoptera frugiperda |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil des Wirkstoffs bzw. des Wirkstoffgemisches mit den angegebenen Mengen Lösungsmittel und Emulgator und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoff- bzw. Wirkstoffgemischgewichtsmenge pro Volumeneinheit Boden, welche in ppm(mg/l) angegebenen wird.

Man füllt Boden in 0.5 1 Töpfe und läßt diese bei 20°C stehen. Sofort nach Ansatz werden je Topf 3 vorgekeimte Maiskörner ausgelegt. Nach Auflaufen der Maiskörner werden Bohrhülsen auf die Töpfe gesetzt. 9 Tage nach dem Ansatz wird der Mais mit den Testinsekten besetzt. Nach weiteren 5 Tagen wird der Abtötungsgrad in % ermittelt. Dabei bedeutet 100%, daß alle Testinsekten abgetötet wurden; 0% bedeutet, daß noch genauso viele Insekten leben wie in der unbehandelten Kontrolle.

Bei diesem Test zeigen z.B. die folgenden Wirkstoffkombinationen gemäß vorliegender Anmeldung eine synergistisch verstärkte Wirksamkeit im Vergleich zu den einzeln angewandten Wirkstoffen:

Tabelle B

**Spodoptera frugiperda Test**

| Wirkstoff/Wirkstoffgemisch | Wirkstoff/Wirkstoffgemisch-konzentration in ppm | Abtötungsgrad in % |
|---|---|---|
| 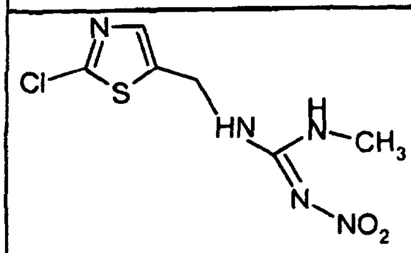 gemäß Formel (I) | 1.25<br>0.60<br>0.30 | 100<br>98<br>50 |
| 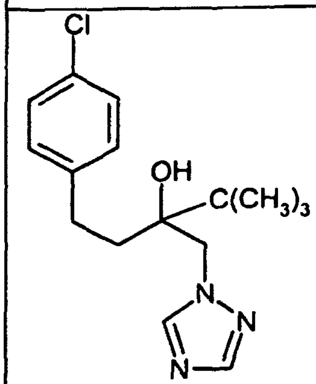 Tebuconazol | 20.00 | 0 |
| 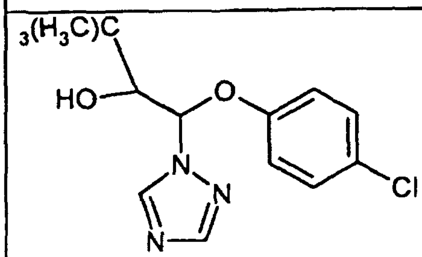 Triadimenol | 20.00 | 0 |
| 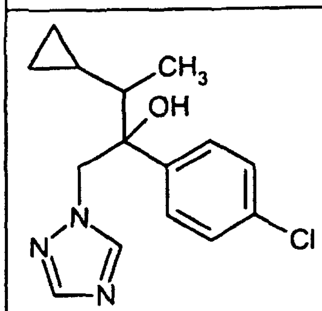 Cypronazol | 20.00 | 0 |

**Tabelle B** (Fortsetzung)

| Wirkstoff/Wirkstoffgemisch | Wirkstoff/Wirkstoffgemisch-konzentration in ppm | Abtötungsgrad in % |
|---|---|---|
| Formel (I) + Tebuconazol | 1.25 + 20.00 | 100 |
| | 0.60 + 20.00 | 80 |
| | 0.30 + 20.00 | 80 |
| Formel (I) + Triadimenol | 1.25 + 20.00 | 100 |
| | 0.60 + 20.00 | 80 |
| | 0.30 + 20.00 | 50 |
| Formel (I) + Cyproconazol | 1.25 + 20.00 | 100 |
| | 0.60 + 20.00 | 50 |
| | 0.30 + 20.00 | 0 |

**Beispiel C**

| **Grenzkonzentrationstest/Wurzelsystemische Wirkung** | |
|---|---|
| Testinsekt | Phaedon cochleariea-Larven |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil des Wirkstoffs bzw. des Wirkstoffgemisches mit den angegebenen Mengen Lösungsmittel und Emulgator und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoff- bzw. Wirkstoffgemischgewichtsmenge pro Volumeneinheit Boden, welche in ppm(mg/l) angegebenen wird.

Man füllt Boden in 250 ml Töpfe und bepflanzt diese mit Kohl (Brassica oleracea). Der Wirkstoff bzw. die Wirkstoffkombination kann so von den Pflanzenwurzeln aus dem Boden aufgenommen und in die Blätter transportiert werden.

Nach 7 Tagen werden die Blätter mit den oben genannten Testtieren besetzt. Nach weiteren 3 Tagen wird der Abtötungsgrad in % ermittelt. Dabei bedeutet 100%, daß alle Testinsekten abgetötet wurden; 0% bedeutet, daß noch genauso viele Insekten leben wie in der unbehandelten Kontrolle.

Bei diesem Test zeigen z.B. die folgenden Wirkstoffkombinationen gemäß vorliegender Anmeldung eine synergistisch verstärkte Wirksamkeit im Vergleich zu den einzeln angewandten Wirkstoffen:

**Tabelle C**

**Phaedon cochleariae Test**

| Wirkstoff/Wirkstoffgemisch | Wirkstoff/Wirkstoffgemisch-konzentration in ppm | Abtötungsgrad in % |
|---|---|---|
| 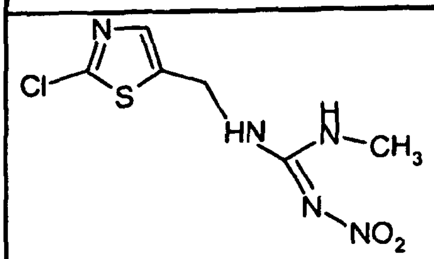 gemäß Formel (I) | 1.25<br>0.60<br>0.30 | 100<br>80<br>50 |
| 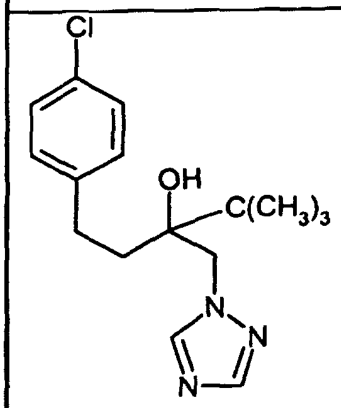 Tebuconazol | 20.00 | 0 |
| 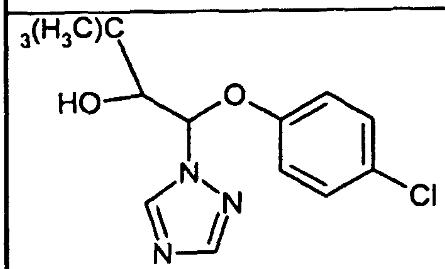 Triadimenol | 20.00 | 0 |
| Formel (I) + Tebuconazol | 1.25 + 20.00<br>0.60 + 20.00<br>0.30 + 20.00 | 100<br>80<br>80 |
| Formel (I) + Triadimenol | 1.25 + 20.00<br>0.60 + 20.00<br>0.30 + 20.00 | 100<br>100<br>100 |

**Beispiel D**

*Berechnungsformel für den Wirkungsgrad einer Kombination aus zwei Wirkstoffen*

Die zu erwartende Wirkung für eine gegebene Kombination zweier Wirkstoffe kann (vgl. Colby, S.R., "Calculating Synergistic and Antagonistic Responses of Herbicide Combinations", Weeds 15, Seiten 20-22, 1967) wie folgt berechnet werden:
Wenn

X den Wirkungsgrad, ausgedrückt in % der unbehandelten Kontrolle, beim Einsatz des Wirkstoffes A in einer Aufwandmenge von m g/ha,

Y den Wirkungsgrad, ausgedrückt in % der unbehandelten Kontrolle, beim Einsatz des Wirkstoffes B in einer Aufwandmenge von n g/ha,

E den Wirkungsgrad, ausgedrückt in % der unbehandelten Kontrolle, beim Einsatz der Wirkstoffe A und B in Aufwandmengen von m und n g/ha bedeutet,

dann ist

$$E = X + Y - \frac{X \times Y}{100}$$

Ist die tatsächliche fungizide Wirkung größer als berechnet, so ist die Kombination in ihrer Wirkung überadditiv, d.h. es liegt ein synergistischer Erffekt vor. In diesem Fall muß der tatsächlich beobachtete Wirkungsgrad größer sein als der aus der oben angeführten Formel errechnete Wert für den erwarteten Wirkungsgrad (E).

| **Phytophthora-Text (Tomate) / protektiv** | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil des Wirkstoffs bzw. des Wirkstoffgemisches mit den angegebenen Mengen Lösungsmittel und Emulgator und verdünnt das Konzentrat mit Wasser auf die gewünschte Konzentration, oder man verdünnt eine handelsübliche Formulierung von Wirkstoff oder Wirkstoffkombination mit Wasser auf die gewünschte Konzentration.

Zur Prüfung auf protektive Wirksamkeit werden junge Pflanzen mit der handelsüblichen Wirkstoffzubereitung in der angegebenen Aufwandmenge besprüht. Nach Antrocknen des Spritzbelages werden die Pflanzen mit einer wäßrigen Sporensuspension von ***Phytophtora infestans*** inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Aus der nachfolgenden Tabelle geht eindeutig hervor, daß die gefundene Wirkung der erfindungsgemäßen Wirkstoffkombinationen größer ist als die berechnete (s.o.), d.h., daß ein synergistischer Effekt vorliegt.

## Tabelle D

Phytophthora Test (Tomate) / protektiv

| Wirkstoff/Wirkstoffgemisch | Wirkstoff/Wirkstoffgemisch in g/ha | Wirkungsgrad in % berechnet/gefunden |
|---|---|---|
| 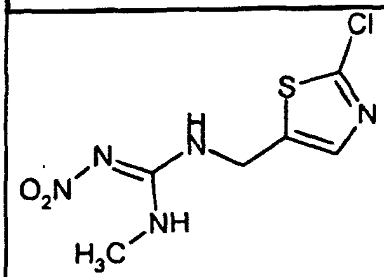 gemäß Formel (I) | 500 | 55 |
| 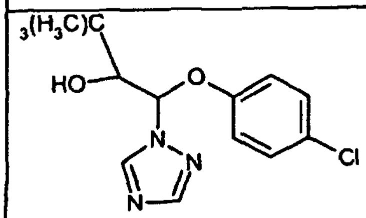 Triadimenol | 500 | 0 |
| Formel (I) + Triadimenol | 500 + 500 | 55/63 |

**Beispiel E**

| Sphaerotheca-Test (Gurke) / protektiv | |
|---|---|
| Lösungsmittel | 47 Gewichtsteile Aceton |
| Emulgator | 3 Gewichtsteile Alkylarylpolyglkyolether |

Zur Herstellung einer zweckmäßigen Wirkstoffzubereitung vermischt man 1 Gewichtsteil des Wirkstoffs bzw. des Wirkstoffgemisches mit den angegebenen Mengen Lösungsmittel und Emulgator und verdünnt das Konzentrat mit Wasser auf die gewünschte Konzentration, oder man verdünnt eine handelsübliche Formulierung von Wirkstoff oder Wirkstoffkombination mit Wasser auf die gewünschte Konzentration.

Zur Prüfung auf protektive Wirksamkeit werden junge Pflanzen mit der handelsüblichen Wirkstoffzubereitung in der angegebenen Aufwandmenge besprüht. Nach Antrocknen des Spritzbelages werden die Pflanzen mit einer wäßrigen Sporensuspension von ***Sphaerotheca fuliginea*** inokuliert. Die Pflanzen werden dann in einer Inkubationskabine bei ca. 20°C und 100 % relativer Luftfeuchtigkeit aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Aus der nachfolgenden Tabelle geht eindeutig hervor, daß die gefundene Wirkung der erfindungsgemäßen Wirkstoffkombinationen größer ist als die berechnete (s.o.), d.h., daß ein synergistischer Effekt vorliegt.

**Tabelle E**

Sphaerotheca-Test (Gurke) / protektiv

| Wirkstoff/Wirkstoffgemisch | Wirkstoff/Wirkstoffgemisch in g/ha | Wirkungsgrad in % berechnet/gefunden |
|---|---|---|
| 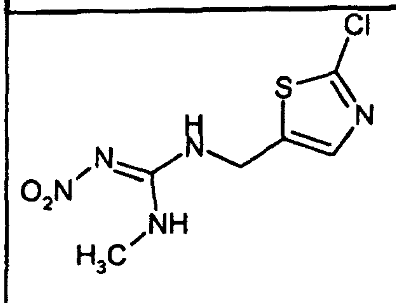 gemäß Formel (I) | 10 | 0 |
| 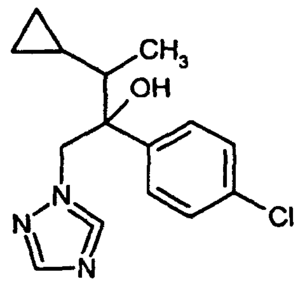 Cyproconazol | 10 | 70 |
| Formel (I) + Cyproconazol | 10 + 10 | 70/80 |

**Beispiel F**

| Botrytis-Test (Bohne) / protektiv | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil des Wirkstoffs bzw. des Wirkstoffgemisches mit den angegebenen Mengen Lösungsmittel und Emulgator und verdünnt das Konzentrat mit Wasser auf die gewünschte Konzentration, oder man verdünnt eine handelsübliche Formulierung von Wirkstoff oder Wirkstoffkombination mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20□C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, daß kein Befall beobachtet wird.

Aus der nachfolgenden Tabelle geht eindeutig hervor, daß die gefundene Wirkung der erfindungsgemäßen

Wirkstoffkombinationen größer ist als die berechnete (s.o.), d.h., daß ein synergistischer Effekt vorliegt.

**Tabelle F**

Borytris-Test (Bohne) / protektiv

| Wirkstoff/Wirkstoffgemisch | Wirkstoff/Wirkstoffgemisch in g/ha | Wirkungsgrad in % berechnet/gefunden |
|---|---|---|
| 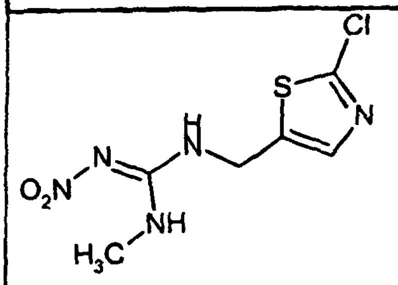 gemäß Formel (I) | 100 | 4 |
| 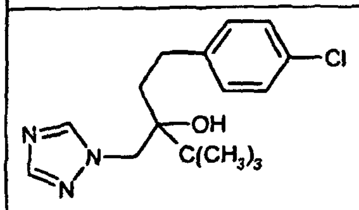 Tebuconazol | 100 | 80 |
| Formel (I) + Tebuconazol | 100 + 100 | 81/94 |

**Beispiel G**

**Fusarium culmorum-Test (Weizen) / Saatgutbehandlung**

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome. Dabei bedeutet 0 % ein Wirkungsgrad, der demjenigen der Kontrolle entspricht, während ein Wirkungsgrad von 100 % bedeutet, daß kein Befall beobachtet wird.

**Tabelle G**

Fusarium culmorun-Test(Weizen)/ Saatgutbehandlung

| Wirkstoff/Wirkstoffgemisch | Wirkstoff/Wirkstoffgemisch in g/ha | Wirkungsgrad in % |
|---|---|---|
| 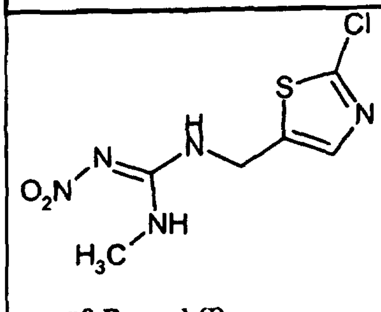 gemäß Formel (I) | 75 | 0 |
| 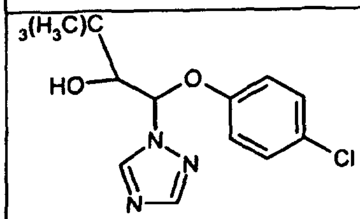 Triadimenol | 75 | 13.5 |
| Formel (I) + Triadimenol | 37.5 + 37.5 | 30.5 |

**Beispiel H**

| Puccinia-Test (Weizen) / protektiv | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff oder Wirkstoffkombination mit den angegebenen Mengen Lösungsmittel und Emulgator und verdünnt das Konzentrat mit Wasser auf die gewünschte Konzentration oder man verdünnt eine handelsübliche Formulierung von Wirkstoff oder Wirkstoffkombination mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von ***Puccinia recondita*** besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

**Tabelle H**

Puccinaria (Weizen)/ protektiv

| Wirkstoff/Wirkstoffgemisch | Wirkstoff/Wirkstoffgemisch in g/ha | Wirkungsgrad in % |
|---|---|---|
| 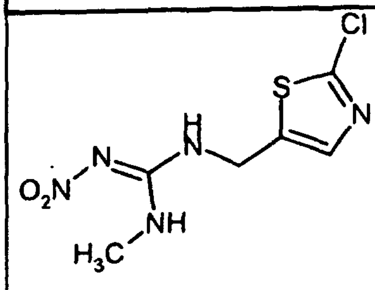 gemäß Formel (I) | 18.75 | 0 |
| 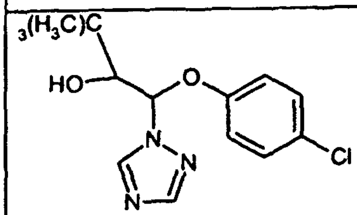 Triadimenol | 18.75 | 13 |
| Formel (I) + Triadimenol | 9.375 + 9.375 | 63 |

**Patentansprüche**

**1.** Mittel enthaltend die Verbindung der Formel (I).

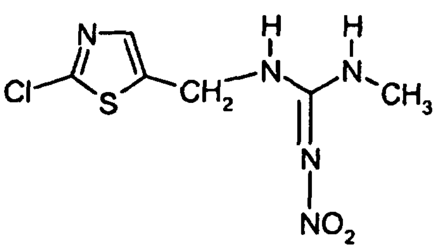

(I)

in Mischung mit einem oder mehreren fungiziden Wirkstoffen aus der Reihe

(1) Azol-Derivate der Formel

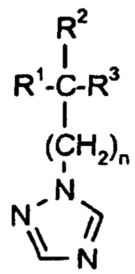

(II)

(II-1) $R^1$ = 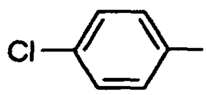 $(CH_2)_2$-, $R^2$ = $-C(CH_3)_3$, $R^3$ = OH, N = 1,

(TEBUCONAZOLE)

(II-3) $R^1$ = 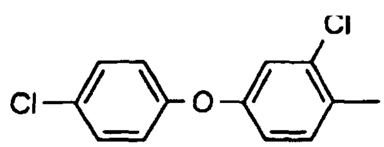 , n = 1,

$R^2$, $R^3$ = $-OCH_2CH(CH_3)O-$,

(DIFENCONAZOLE)

(II-4) $R^1$ = 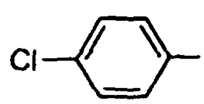 , $R^2$ = 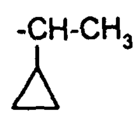 , $R^3$ = -OH, n = 1,

(CYPROCONAZOLE)

(II-5) $R^1$ = 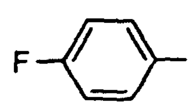 , $R^2$ = 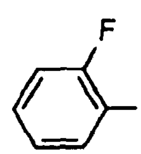 , $R^3$ = OH, n = 1,

(FLUTRIAFOL)

(II-6) $R^3$ = 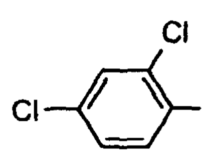 , $R^4$ = $-(CH_2)_3CH_3$, $R^5$ = OH, n = 1,

(HEXACONAZOLE)

(II-7) $R^1 =$ 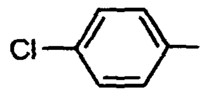 , $R^2 = -(CH_2)_3CH_3$, $R^3 = CN$, $n = 1$,

(MYCLOBUTANIL)

(II-8) $R^1 =$ 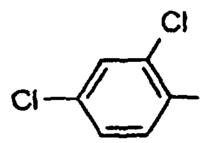 , $R^2 = -(CH_2)_2CH_3$, $R^3 = H$, $n = 1$,

(PENCONAZOLE)

(II-9) $R^1 =$ 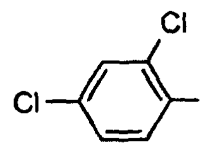 , $R^2, R^3 =$ 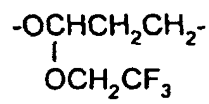 , $n = 1$,

(FURCONAZOLE)

(II-10) $R^1 =$ 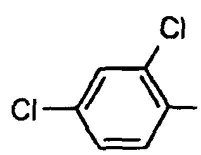 , $R^2, R^3 =$ 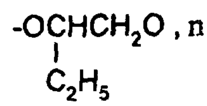 , $n = 1$,

(ETACONAZOLE)

(II-11) $R^1 =$ 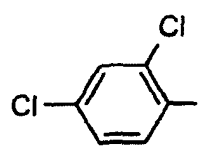 , $R^2, R^3 =$ 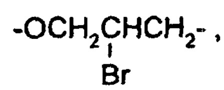 , $n = 1$,

(BROMUCONAZOLE)

(II-12) $R^1 =$ 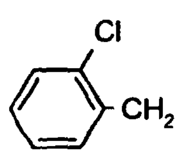 , $R^2 =$ 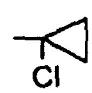 , $R^3 = OH$, $n = 1$,

(II-13) $R^1$ = 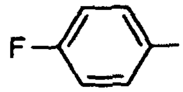, $R^2$, $R^3$ = 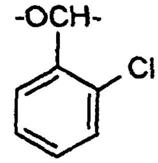, n = 1,

(II-14) $R^1$ = 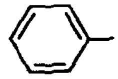, $R^2$ = 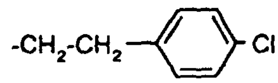, n = 1, $R^3$ = CN,

(FENBUCONAZOLE)

(II-15) $R^1$ = 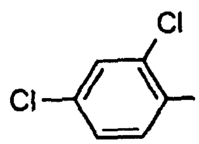, $R^2 = CH_2OCF_2CHF_2$, $R^3$ = H, n = 1,

(TETRACONAZOLE)

(II-16) $R^1$ = 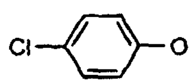, $R^2 = -CH(OH)-C(CH_3)_3$, n = 0, $R^3$ = H,

(TRIADIMENOL)

(II-17) $R^1$ = 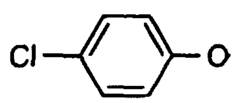, $R^2 = -CO-C(CH_3)_3$, n = 0, $R^3$ = H,

(TRIADIMEFON)

(II-19) $R^1$ = 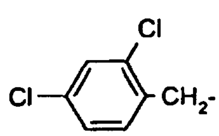, $R^2 = -CH(OH)-C(CH_3)_3$, n = 0,

$R^3$ = H,

(DICLOBUTRAZOL)

(II-20) $R^1$ und $R^2$ = 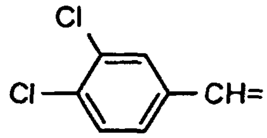 , $R^3$ = 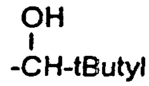 , n = 0.

(DINICONAZOL)

**2.** Mittel gemäß Anspruch 1, **dadurch gekennzeichnet, dass** in den Wirkstoffkombinationen auf 1 Gewichtsteil an Wirkstoff der Formel (I) 0,1 bis 10 Gew.-Teile an mindestens einem fungiziden Wirkstoff entfallen.

**3.** Verfahren zur Bekämpfung von Pilzen und Insekten, **dadurch gekennzeichnet, dass** man Wirkstoffkombinationen gemäß Anspruch 1 auf die Pilze, Insekten und/oder deren Lebensraum einwirken lässt.

**4.** Verwendung von Wirkstoffkombinationen gemäß Anspruch 1 zur Bekämpfung von Pilzen und Insekten.

**5.** Verfahren zur Herstellung von Mitteln gemäß Anspruch 1, **dadurch gekennzeichnet, dass** man Wirkstoffkombinationen gemäß Anspruch 1 mit Streckmitteln und/oder oberflächenaktiven Stoffen vermischt.

**Claims**

**1.** Compositions containing the compound of the formula (I)

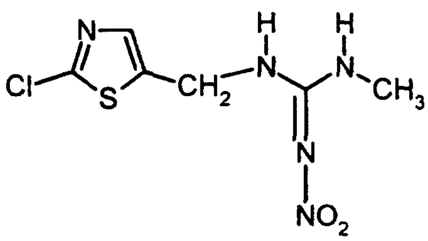

(I)

in a mixture with one or more fungicidal active compounds from the group consisting of

(1) azole derivatives of the formula

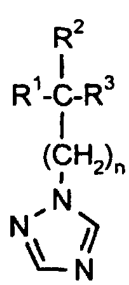

(II)

(II-1) $R^1$ = 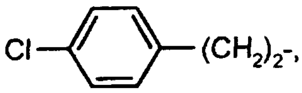, $R^2 = -C(CH_3)_3$, $R^3$ = OH, N = 1,

(TEBUCONAZOLE)

(II-3) $R^1$ = 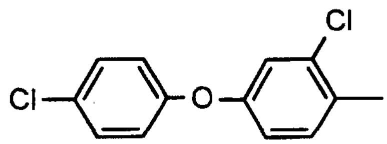, n = 1,

$R^2$, $R^3 = -OCH_2CH(CH_3)O-$,

(DIFENCONAZOLE)

(II-4) $R^1$ = 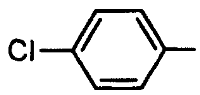, $R^2$ = 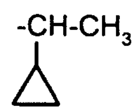, $R^3$ = -OH, n = 1,

(CYPROCONAZOLE)

(II-5) $R^1$ = 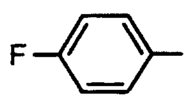, $R^2$ = 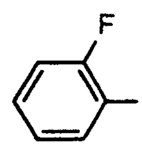, $R^3$ = OH, n = 1,

(FLUTRIAFOL)

(II-6) $R^3$ = 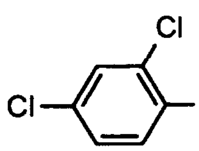, $R^4 = -(CH_2)_3CH_3$, $R^5$ = OH, n = 1,

(HEXACONAZOLE)

(II-7) $R^1$ = 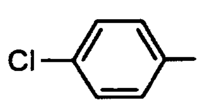, $R^2 = -(CH_2)_3CH_3$, $R^3$ = CN, n = 1,

(MYCLOBUTANIL)

(II-8) $R^1$ = 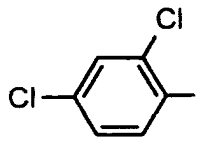, $R^2$ = $-(CH_2)_2CH_3$, $R^3$ = H, n = 1,

(PENCONAZOLE)

(II-9) $R^1$ = 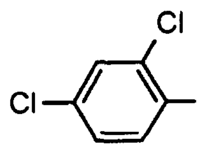, $R^2$, $R^3$ = 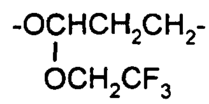, n = 1,

(FURCONAZOLE)

(II-10) $R^1$ = 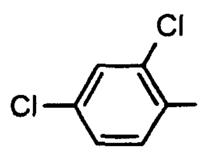, $R^2$, $R^3$ = 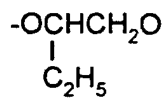, n = 1,

(ETACONAZOLE)

(II-11) $R^1$ = 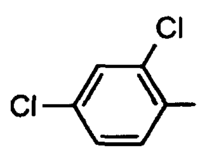, $R^2$, $R^3$ = 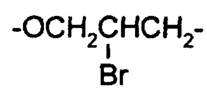, n = 1,

(BROMUCONAZOLE)

(II-12) $R^1$ = 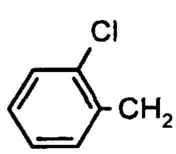, $R^2$ = 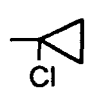, $R^3$ = OH, n = 1,

(II-13) $R^1$ = 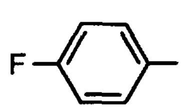, $R^2$, $R^3$ = 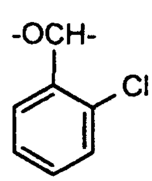, n = 1,

(II-14) $R^1$ = 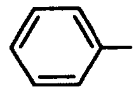, $R^2$ = 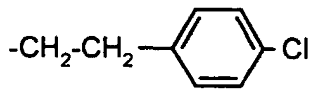, n = 1, $R^3$ = CN,

(FENBUCONAZOLE)

(II-15) $R^1$ = 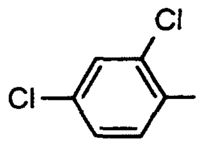, $R^2 = CH_2OCF_2CHF_2$, $R^3$ = H, n = 1,

(TETRACONAZOLE)

(II-16) $R^1$ = 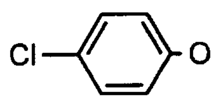, $R^2 = -CH(OH)-C(CH_3)_3$, n = 0, $R^3$ = H,

(TRIADIMENOL)

(II-17) $R^1$ = 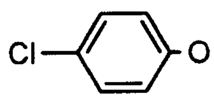, $R^2 = -CO-C(CH_3)_3$, n = 0, $R^3$ = H,

(TRIADIMEFON)

(II-19) $R^1$ = 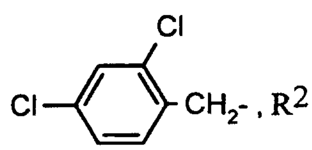, $R^2 = -CH(OH)-C(CH_3)_3$, n = 0, $R^3$ = H,

(DICLOBUTRAZOLE)

(II-20) $R^1$ and $R^2$ = 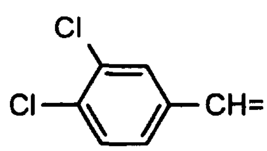, $R^3$ = 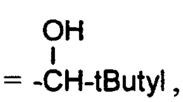, n = 0,

(DINICONAZOLE)

2. Compositions according to Claim 1, **characterized in that** the active compound combinations contain from 0.1 to 10 parts by weight of at least one fungicidal active compound per part by weight of active compound of the formula (I).

3. Process for controlling fungi and insects, **characterized in that** active compound combinations according to Claim 1 are allowed to act on the fungi, insects and/or their habitat.

**4.** Use of active compound combinations according to Claim 1 for controlling fungi and insects.

**5.** Process for preparing compositions according to Claim 1, **characterized in that** active compound combinations according to Claim 1 are mixed with extenders and/or surfactants.

**Revendications**

**1.** Agents contenant le composé de formule (I)

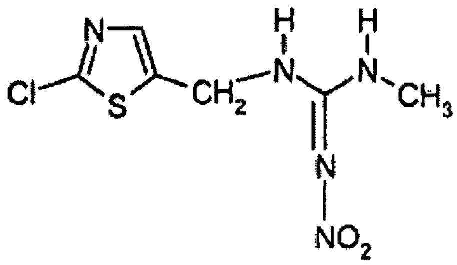

(I)

mélangé avec un ou plusieurs principes actifs fongicides de la série :

(1) dérivés d'azoles de formule

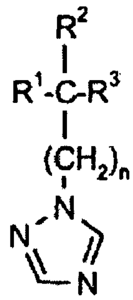

(II)

(II-1) $R^1$ = 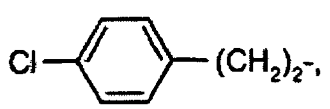, $R^2 = -C(CH_3)_3$, $R^3 = OH$, N = 1,

(TEBUCONAZOLE)

(II-3) $R^1$ = 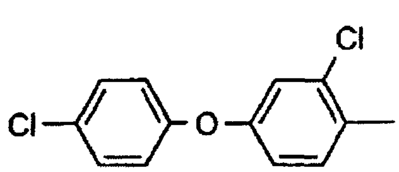, n = 1,

$R^2$, $R^3 = -OCH_2CH(CH_3)O-$,

(DIFENCONAZOLE)

(II-4) $R^1$ = 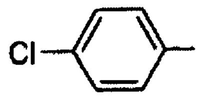 , $R^2$ = 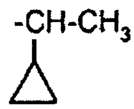 , $R^3$ = -OH, n = 1,

(CYPROCONAZOLE)

(II-5) $R^1$ = 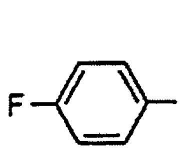 , $R^2$ = 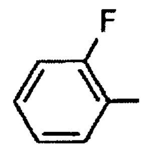 , $R^3$ = OH, n = 1,

(FLUTRIAFOL)

(II-6) $R^3$ = 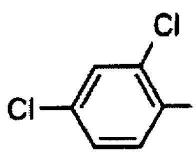 , $R^4$ = $-(CH_2)_3CH_3$, $R^5$ = OH, n = 1,

(HEXACONAZOLE)

(II-7) $R^1$ = 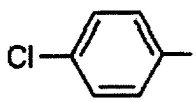 , $R^2$ = $-(CH_2)_3CH_3$, $R^3$ = CN, n = 1,

(MYCLOBUTANIL)

(II-8) $R^1$ = 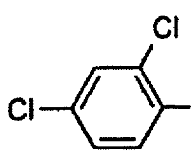 , $R^2$ = $-(CH_2)_2CH_3$, $R^3$ = H, n = 1,

(PENCONAZOLE)

(II-9) $R^1$ = 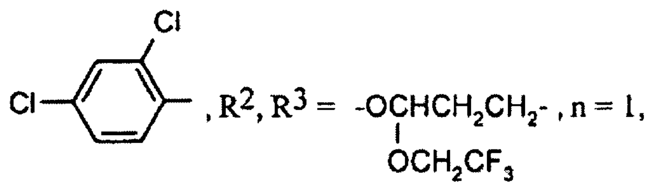

(FURCONAZOLE)

(II-10) $R^1$ = 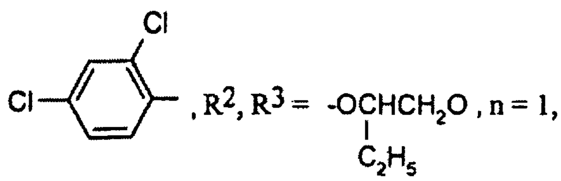

(ETACONAZOLE)

(II-11) $R^1$ = 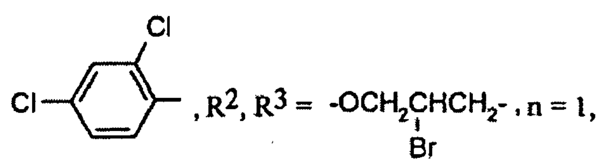

(BROMUCONAZOLE)

(II-12) $R^1$ = 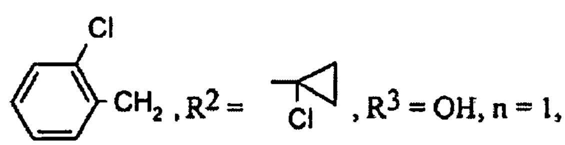

(II-13) $R^1$ = 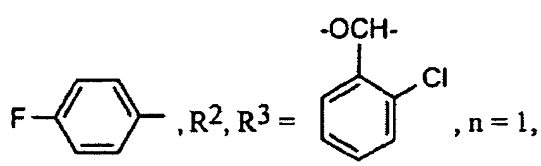

(II-14) $R^1$ = 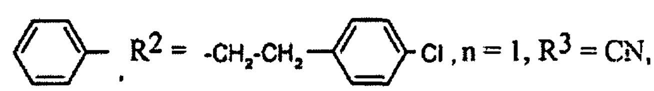

(FENBUCONAZOLE)

(II-15) $R^1$ = 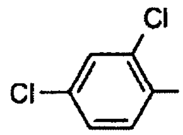, $R^2 = CH_2OCF_2CHF_2$, $R^3$ = H, n = 1,

(TETRACONAZOLE)

(II-16) $R^1$ = 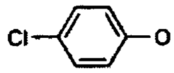, $R^2$ = -CH(OH)-C$(CH_3)_3$, n = 0, $R^3$ = H,

(TRIADIMENOL)

(II-17) $R^1$ = 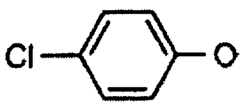, $R^2$ = -CO-C$(CH_3)_3$, n = 0, $R^3$ = H,

(TRIADIMEFON)

(II-19) $R^1$ = 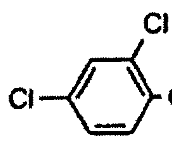 $CH_2$-, $R^2$ = -CH(OH)-C$(CH_3)_3$, n = 0,

$R^3$ = H,

(DICLOBUTRAZOLE)

(II-20) $R^1$ et $R^2$ = 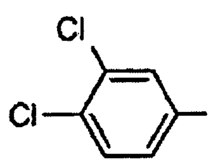 CH=, $R^3$ = -CH-tertio-butyle (OH), n = 0.

(DINICONAZOLE)

2. Agents selon la revendication 1, **caractérisés en ce que** les combinaisons de principes actifs contiennent de 0,1 à 10 parties en poids d'au moins un principe actif fongicide pour 1 partie en poids de principe actif de formule (I).

3. Procédé pour lutter contre les champignons et les insectes, **caractérisé en ce qu'**on laisse agir des combinaisons de principes actifs selon la revendication 1 sur les champignons, les insectes et/ou sur leur biotope.

4. Utilisation de combinaisons de principes actifs selon la revendication 1 pour lutter contre les champignons et les

insectes.

5. Procédé de préparation d'agents selon la revendication 1, **caractérisé en ce qu'**on mélange des combinaisons de principes actifs selon la revendication 1 avec des agents d'allongement et/ou des agents tensioactifs.